Europäisches Patentamt

European Patent Office

Office européen des brevets

(11) Publication number: **0 194 880**
**B1**

(12) **EUROPEAN PATENT SPECIFICATION**

(45) Date of publication of patent specification: **30.01.91**

(51) Int. Cl.⁵: **A 61 K 9/14,** A 61 K 47/00

(21) Application number: **86301797.6**

(22) Date of filing: **12.03.86**

(54) **Multivitamin preparation.**

(30) Priority: **12.03.85 JP 48880/85**

(43) Date of publication of application:
**17.09.86 Bulletin 86/38**

(45) Publication of the grant of the patent:
**30.01.91 Bulletin 91/05**

(84) Designated Contracting States:
**AT BE CH DE FR GB IT LI LU NL SE**

(56) References cited:
**DE-A-2 441 761**
**FR-A-2 340 981**
**GB-A- 946 086**
**GB-A-1 126 289**
**GB-A-1 225 979**
**US-A-4 011 661**

**CHEMICAL ABSTRACTS, vol. 97, no. 4, 26th July
1982, page 306, no. 28541d, Columbus, Ohio,
US; H. HSU et al.: "Formulation studies on
stable vitamin B-C complex parenteral solution
by accelerated stability analysis".**

(73) Proprietor: **SANKYO COMPANY LIMITED
No. 1-6, 3-chome Nihonbashi Honcho Chuo-ku
Tokyo (JP)**

(72) Inventor: **Ueda, Seigo Sankyo Product
Development Lab.
No. 2-58, 1-chome, Hiromachi Shinagawa-ku
Tokyo 140 (JP)**
Inventor: **Kusai, Akira Sankyo Product
Development Lab.
No. 2-58, 1-chome, Hiromachi Shinagawa-ku
Tokyo 140 (JP)**
Inventor: **Shiokari, Takashi Sankyo Product
Development Lab.
No. 2-58, 1-chome, Hiromachi Shinagawa-ku
Tokyo 140 (JP)**
Inventor: **Fukami, Masaharu, Dr. Sankyo
Product Develop. Lab.
No. 2-58, 1-chome, Hiromachi Shinagawa-ku
Tokyo 140 (JP)**

(74) Representative: **Gibson, Christian John Robert
MARKS & CLERK 57/60 Lincoln's Inn Fields
London WC2A 3LS (GB)**

## Description

The present invention relates to a novel multivitamin preparation.

It is frequently convenient to administer a plurality of vitamins together in one preparation, for therapeutic or nutritional purposes. Multivitamin preparations intended for parenteral administration may be presented in a lyophilized state, for later reconstitution by the addition of water for injections or a suitable infusion liquid, immediately before use. Some of the vitamins used in such preparations are water-soluble whilst others are fat-soluble, and some of them are liquid at ambient temperature. The conventional method of obtaining a lyophilized product from such a vitamin mixture has involved the use of a surfactant (e.g. a polysorbate surfactant) to solubilize the fat-soluble vitamins in water, and the addition of an excipient such as dextran to the mixed vitamin solution prior to lyophilization.

However, it has been found that certain disadvantages arise from the use of excipients such as dextran. Multivitamin preparations containing such excipients are less stable in storage, as can be demonstrated by their deterioration during accelerated storage tests at elevated temperature; and in experiments with rats these preparations have exhibited undesirable side effects such as hypertrophy of the spleen. The cause of these undesirable properties is not known, but it is thought that they may arise from an interaction between the excipient, surfactant and some fat-soluble vitamins.

British Patent No. 946086 and Chemical Abstracts 97:28541d both disclose excipient free solutions of fat-soluble vitamins for parenteral injection using the condensation product of castor oil and ethylene oxide. Such solutions, free from dextran or other similar excipient, can eliminate or mitigate the above-mentioned side-effects but, when lyophilised, are still subject to deterioration.

The registered Trade marks as used in the following are acknowledged as such.

The present invention provides a lyophilized excipient-free multivitamin preparation characterised by the absence of any excipient and by the presence of: a hydrogenated castor oil surfactant; a polyoxypropylene condensate surfactant, preferably polyoxalene; and a phospholipid, preferably a lecithin, lecithin derivative or lecithin principal component or sphingomyelin.

The multivitamin preparation of the invention is superior to the conventional excipient-containing type of preparation both as regards stability at elevated temperatures and as regards a reduced incidence of side-effects such as hypertrophy of the spleen. The improved stability at elevated temperatures is marked when using both polyoxypropylene condensate nonionic surfactant and phospholipid, in addition to the hydrogenated castor oil type surfactant, but not with the hydrogenated castor oil type surfactant in combination with only one.

The multivitamin preparation of the present invention may comprise any of the conventional vitamin components. Examples of fat-soluble vitamins include vitamins A, $D_3$, E, and $K_2$. Examples of water-soluble vitamins include vitamins $B_1$, $B_2$, $B_6$, $B_{12}$, nicotinamide, folic acid, panthenol, and vitamins C and H.

The hydrogenated castor oil surfactants used in the present invention are non-ionic polyoxyalkylene hydrogenated castor oil derivatives, such as "Polyoxy 40 Hydrogenated Castor Oil" (U.S. Pharmacopoeia), which can be obtained by the addition polymerization of an alkylene oxide, typically ethylene oxide, or addition copolymerization of ethylene oxide and propylene oxide, to hydrogenated castor oil. About 30 to 150 moles, preferably about 40 to 120 moles, of said alkylene oxide may conveniently be employed for the addition polymerization per mole of hydrogenated castor oil; and, most preferably, about 50 to 100 moles of ethylene oxide are employed for the addition polymerization per mole of hydrogenated castor oil. Commercially available examples of such surfactants are those produced by the Nikko Chemicals Co. under the designations HCO 40, HCO 50, HCO 60, HCO 80 and HCO 100, which respectively have HLB values of 11.8, 13.4, 14.1, 15.4 and 16.5, and in which the numbers 40, 50, 60, 80 and 100 indicate the approximate number of moles of ethylene oxide per mole of hydrogenated castor oil.

The polyoxypropylene condensate type surfactant may be any of those non-ionic surfactants formed by addition polymerization of ethylene oxide with propylene glycol as a hydrophobic group. Examples of these are the poly(oxyethylene)-poly(oxypropylene)-poly(oxyethylene) polymers such as polyoxalene. Commercially available examples of such surfactants include those produced by Asahi Denka Kogyo K.K. under the designations Pluronic F-68 and Pluronic F-88.

Examples of suitable phospholipids include: yolk lecithin; soybean lecithin; dipalmitoylphosphatidyl choline (DPPC), distearoylphosphatidyl choline (DSPC), dipalmitoylphosphatidyl ethanolamine (DPPE) and dipalmitoxylphosphatidyl inositol (DPPI), each of which is a principal component of lecithin; lysolecithin, which is a hydrolysate of lecithin; lecithin hydroxide, obtained by hydroxylation of the unsaturated portion of the lecithin; and sphingomyelin.

In practising the present invention, the fat-soluble vitamins may be solubilized in water, suitably at a temperature of around 50—80°C, by using about 2—10 parts by weight of hydrogenated castor oil surfactant per part by weight of vitamin. The optional polyoxypropylene condensate type nonionic surfactant is suitably added in an amount of 5—40%, preferably 10—25%, by weight based on the weight of the vitamin. The optionally used phospholipid is suitably added in an amount of 1—60%, preferably 2—30%, by weight based on the weight of vitamin.

The water-soluble vitamin components are suitably dissolved in water at around 10—20°C and the pH of the solution is adjusted to a value of about 5—6 by adding a base such as sodium hydrogen carbonate.

The respective solutions of fat-soluble and water-soluble vitamins are then mixed and the volume is

2

adjusted by adding water for injections, suitably to give a concentration of the vitamins therein of around 1—10% by weight. The resulting solution can then be portioned into vials or other suitable containers and lyophilized by conventional techniques.

In order to use the lyophilized multivitamin preparation of the invention, it will normally be reconstituted in a pharmaceutically acceptable vehicle suitable for parenteral administration, such as water for injections or an infusion liquid (e.g. physiological saline, a glucose solution or an intravenous hyperalimentation solution). The reconstituted preparation may then be administered in any conventional manner, such as by injection or by an intravenous drip.

The following Examples 1—17 illustrate the invention in further detail. Examples 1—5 illustrate formulations comprising hydrogenated castor oil surfactant but lacking one or both of the other components. Comparative Examples 1 and 2 describe preparations outside the scope of the invention, for purposes of comparison. Experiments 1 and 2 demonstrate the properties of the preparations made in accordance with the Examples and Comparative Examples. The "HCO" surfactants used in the Examples were all hydrogenated castor oil surfactants of the type described hereinabove and manufactured by the Nikko Chemicals Co.

Example 1

A multivitamin preparation was made using the vitamins shown in Table 1. The amount indicated for each vitamin is that used per 5 ml portion of solution, and the total amount used was sufficient for 200 such portions.

The fat-soluble vitamins were solubilized in water for injections by using 100 mg of HCO 60 surfactants per portion (i.e. a total of 20 g). The water-soluble vitamins were dissolved in water for injections, and the pH of the solution was adjusted to 5—6 with sodium hydrogencarbonate. The two solutions were mixed together and the total volume was made up to 1 litre with water for injections. The solution was divided between 20 vials and freeze-dried to obtain the lyophilized multivitamin preparation.

TABLE 1

| Vitamin | Amount (per) 5 ml portion) |
|---|---|
| Vitamin $B_1$ (thiamine hydrochloride) | 15.0 mg |
| Vitamin $B_2$ (sodium riboflavine phosphate) | 5.0 mg |
| Vitamin $B_6$ (pyridoxine hydrochloride) | 7.5 mg |
| Vitamin $B_{12}$ (cyanocobalamine) | 0.5 mg |
| Nicotinamide | 60.0 mg |
| Panthenol | 5.0 mg |
| Folic acid (pteroylmonoglutamic acid) | 0.4 mg |
| Vitamin C (ascorbic acid) | 200.0 mg |
| Vitamin H (biotin) | 0.2 mg |
| Vitamin A (retinol palmitate) | 5000 I.U. |
| Vitamin $D_3$ (cholecalciferol) | 400 I.U. |
| Vitamin E (tocopherol acetate) | 5.0 mg |
| Vitamin $K_2$ (menatetrenone) | 4.0 mg |

Example 2

A multivitamin preparation was made as in Example 1, except that 70 mg of HCO 50 and 20 mg of Pluronic F-68 surfactants were used in place of the 100 mg of HCO 60 surfactant.

# EP 0 194 880 B1

### Example 3
A multivitamin preparation was made as in Example 1, except that 80 mg of HCO 100 surfactant was used in place of the 100 mg of HCO 60 surfactant.

### Example 4
A multivitamin preparation was made as in Example 1, except that 80 mg of HCO 60 and 20 mg of Pluronic F-68 surfactants were used in place of the 100 mg of HCO 60 surfactant.

### Example 5
A multivitamin preparation was made as in Example 1, except that 100 mg of HCO 60 surfactant and 10 mg of yolk lecithin (Kewpie PL-100) were used in place of the 100 mg of HCO 60 surfactant.

### Example 6
A multivitamin preparation was made as in Example 1, except that 80 mg of HCO 60 surfactant, 20 mg of Pluronic F-68 surfactant and 10 mg of yolk lecithin (Kewpie PL-100) were used in place of the 100 mg of HCO 60 surfactant.

### Examples 7 to 17
Example 6 was repeated, but using the following mixtures of hydrogenated castor oil type surfactant polyoxypropylene condensate type nonionic surfactant and phospholipid:—

| Example No. | |
|---|---|
| 7. | HCO 50, 80 mg; Pluronic F-68, 20 mg; Yolk lecithin (Kewpie PL-100), 10 mg. |
| 8. | HCO 100, 80 mg; Pluronic F-88, 20 mg; Yolk lecithin (Kewpie PL-100), 10 mg. |
| 9. | HCO 60, 80 mg; Pluronic F-68, 20 mg; Yolk lecithin (Kewpie PL-100), 10 mg. |
| 10. | HCO 60, 80 mg; Pluronic F-68, 20 mg; Soybean lecithin (Nikkol Lecinol 30), 10 mg. |
| 11. | HCO 60, 80 mg; Pluronic F-68, 20 mg; Lysolecithin (Nikkol Lecinol LL-20), 10 mg. |
| 12. | HCO 60, 80 mg; Pluronic F-68, 20 mg; Lecithin hydroxide (Nikkol Lecinol SH), 10 mg. |
| 13. | HCO 60, 80 mg; Pluronic F-68, 20 mg; DPPC (produced by Sigma Co.), 10 mg. |
| 14. | HCO 60, 80 mg; Pluronic F-68, 20 mg; DSPC (produced by Sigma Co.), 10 mg. |
| 15. | HCO 60, 80 mg; Pluronic F-68, 20 mg; DPPE (produced by Sigma Co.), 10 mg. |
| 16. | HCO 60, 80 mg; Pluronic F-68, 20 mg; DPPI (produced by Sigma Co.), 10 mg. |
| 17. | HCO 60, 80 mg; Pluronic F-68, 20 mg; Sphingomyelin (produced by Sigma Co.), 10 mg. |

### Comparative Example 1
The procedure of Example 1 was repeated, except that 100 mg of polysorbate 80 surfactant ("Tween 80" produced by KAO Atlas Powder Co.) were used to solubilize the fat-soluble vitamins in place of the 100 mg of HCO 60 surfactant. When the final solution was subjected to freeze-drying, a gum-like substance was obtained and it was not possible to produce a satisfactory lyophilized preparation.

4

Comparative Example 2

The procedure of Example 1 was repeated as far as the preparation of the respective solutions of the fat-soluble and water-soluble vitamins. After mixing together these two solutions, Dextran 40 was added as excipient (Japanese Pharmacopoeia, produced by Meito Sangyo K.K.) in an amount of 400 mg per 5 ml portion (i.e. a total of 80 g). The total volume of the solution was made up to 1 litre, as before, with water for injections, and 5 ml portions of the solution filled into vials and lyophilized.

Experiment 1

Each of the freeze-dried products obtained in Examples 1, 2, 3 and 6 and Comparative Example 2 was redissolved in 5 ml of water for injections. Each of the solutions was administered by intravenous injection for a period of one week to male Fischer rats at a dose of 20 ml/day/kg bodyweight (4 rats per solution, 7—8 weeks old, average weight at beginning of experiment $17.65 \pm 2.5$ g). The rats were sacrificed at the end of the week, and their spleens were removed and weighed.

The results of the experiment are shown in Table 2, from which it will be seen that spleens taken from the animals treated with the excipient-containing preparation of Comparative Example 2 weighed nearly twice as much as those taken from the animals treated with the preparations containing no excipient (Examples 1, 2, 3 and 6).

TABLE 2

| Preparation | Average weight of spleen |
|---|---|
| Example 1 | $0.51 \pm 0.01$ g |
| Example 2 | $0.49 \pm 0.01$ g |
| Example 3 | $0.52 \pm 0.01$ g |
| Example 6 | $0.50 \pm 0.01$ g |
| Comparative Example 2 | $0.95 \pm 0.01$ g |

Experiment 2

The free-dried products of Examples 1 and 4 to 17 and Comparative Example 2 were stored for 8 weeks at 50°C. Samples from each were taken after 4 and 8 weeks and reconstituted with 10 ml of water for injections. Each reconstituted solution was poured into a clean test tube and examined visually by a skilled observer, against white and black backgrounds, under a white light source of 100 W at an illumination of about 5,000 lux. Each solution was assessed in this way for the presence of suspended foreign matter. The results are shown in Table 3.

As will be seen from Table 3, the appearance of the reconstituted excipient-containing preparation of Comparative Example 2 was markedly inferior to that of the reconstituted preparations of the invention. Also, among the preparations of the invention, there was less deterioration in those preparations containing the hydrogenated castor oil type surfactant with both of the optional components, as compared with those containing neither or only one of the two optional components.

TABLE 3

| Example No. | Storage period at 50°C | | |
|---|---|---|---|
| | 0 week | 4 weeks | 8 weeks |
| 1 | ± | ± to + | + to ++ |
| 4 | ± | ± to + | + to ++ |
| 5 | ± | ± to + | + to ++ |
| 6 | – to ± | – to ± | – to ± |
| 7 | – to ± | – to ± | – to ± |
| 8 | – to ± | – to ± | – to ± |
| 9 | – to ± | – to ± | – to ± |
| 10 | – to ± | – to ± | – to ± |
| 11 | – to ± | – to ± | – to ± |
| 12 | – to ± | – to ± | – to ± |
| 13 | – to ± | – to ± | – to ± |
| 14 | – to ± | – to ± | – to ± |
| 15 | – to ± | – to ± | – to ± |
| 16 | – to ± | – to ± | – to ± |
| 17 | – to ± | – to ± | – to ± |
| Comparative Example 2 | + | ++ | ++ |

*Key to results in Table 3:*
Increasing amounts of suspended foreign matter observed in the range from:
   – no foreign matter observed
to
   ++ suspended foreign matter readily observed.

**Claims**

1. A lyophilized excipient-free multivitamin preparation characterised by the absence of any excipient and by the presence of: a hydrogenated castor oil surfactant; a polyoxypropylene condensate surfactant, preferably polyoxalene; and a phospholipid, preferably a lecithin, lecithin derivative or lecithin principal component or sphingomyelin.

2. A multivitamin preparation as claimed in Claim 1, in which said hydrogenated castor oil surfactant is one formed by the addition polymerisation of from about 30 to about 150 moles of alkylene oxide per mole of hydrogenated castor oil.

3. A multivitamin preparation as claimed in Claim 2, in which said hydrogenated castor oil surfactant is one formed by the addition polymerisation of from about 40 to about 120 moles of alkylene oxide per mole of hydrogenated castor oil.

4. A multivitamin preparation as claimed in Claim 3, in which said hydrogenated castor oil surfactant is one formed by the addition polymerization of from about 50 to about 100 moles of ethylene oxide per mole of hydrogenated castor oil.

5. A multivitamin preparation according to any preceding claim, wherein the phospholipid is selected from: yolk lecithin; soybean lecithin; dipalmitoylphosphatidyl choline (DPPC); distearoylphosphatidyl choline (DSPC); dipalmitoylphosphatidyl ethanolamine (DPPE); dipalmitoylphosphatidyl inositol (DPPI); lysolecithin; lecithin hydroxide; and sphingomyelin.

6. A method of preparing an excipient-free lyophilized multivitamin preparation, which comprises the steps of: solubilizing in water at least one fat-soluble vitamin by means of a hydrogenated castor oil surfactant, a polyoxypropylene condensate surfactant, preferably polyoxalene, and a phospholipid, preferably a lecithin, lecithin derivative or lecithin principal component or sphingomyelin, to obtain a first solution; dissolving in water at least one water-soluble vitamin to obtain a second solution; mixing together the said first and second solutions; and freeze-drying the resulting mixed solution to obtain a lyophilized product.

7. A method as claimed in Claim 6, wherein the hydrogenated castor oil surfactant and/or polyoxypropylene condensate surfactant and/or phospholipid are as defined in any of claims 2 to 5.

8. A method of preparing an excipient-free multivitamin preparation suitable for parenteral administration, which comprises the steps of: solubilizing in water at least one fat-soluble vitamin by means of a hydrogenated castor oil surfactant, a polyoxypropylene condensate surfactant, preferably polyoxalene, and a phospholipid, preferably a lecithin, lecithin derivative or lecithin principal component or sphingomyelin, to obtain a first solution; dissolving in water at least one water-soluble vitamin to obtain a second solution; mixing together the said first and second solutions; freeze-drying the resulting mixed solution to obtain a lyophilized product; and reconstituting said lyophilized product in a liquid which is acceptable for parenteral administration.

9. A method as claimed in Claim 8, wherein the hydrogenated castor oil surfactant and/or polyoxypropylene condensate surfactant and/or phospholipid are as defined in any of claims 2 to 5.

10. An excipient-free multivitamin preparation comprising a hydrogenated castor oil surfactant and characterised by the presence of: a polyoxypropylene condensate surfactant, preferably polyoxalene; and a phospholipid, preferably a lecithin, lecithin derivative or lecithin principal component or sphingomyelin.

## Patentansprüche

1. Ein gefriergetrocknetes, exzipientiafreies Multivitaminpräparat, gekennzeichnet durch die Abwesenheit eines jeglichen Exzipiens und durch die Anwesenheit eines oberflächenaktiven Stoffes auf Basis von hydriertem Rhizinusöl, eines oberflächenaktiven Stoffes, vorzugsweise Polyoxalene, auf Basis eines Polyoxypropylenkondensats und eines Phospholipids, vorzugsweise ein Lecithin, ein Lecithinderivat oder einem Lecithinhauptbestandteil oder Sphingomyelin.

2. Multivitaminpräparat nach Anspruch 1, in welchem der oberflächenaktive Stoff auf Basis von hydriertem Rhizinusöl ein durch Additionspolymerisation von etwa 30 bis etwa 150 Mol eines Alkylenoxids je Mol hydrierten Rhizinusöls hergestellter oberflächenaktiver Stoff ist.

3. Multivitaminpräparat nach Anspruch 2, in welchem der oberflächenaktive Stoff auf Basis von hydriertem Rhizinusöl ein durch Additionspolymerisation von etwa 40 bis etwa 120 Mol eines Alkylenoxids je Mol hydrierten Rhizinusöls hergestellter oberflächenaktiver Stoff ist.

4. Multivitaminpräparat nach Anspruch 3, in welchem der oberflächenaktive Stoff auf Basis von hydriertem Rhizinusöl ein durch Additionspolymerisation von etwa 50 bis etwa 100 Mol Äthylenoxid je Mol hydrierten Rhizinusöls hergestellter oberflächenaktiver Stoff ist.

5. Multivitaminpräparat nach irgendeinem vorhergehenden Anspruch, worin das Phospholipid aus Molkelecithin, Sojabohnenlecithin, Dipalmitoylphosphatidylcholin (DPPC), Distearoylphosphatidylcholin (DSPC), Dipalmitoylphosphatidyläthanolamin (DPPE), Dipalmitoylphosphatidylinosit (DPPI), Lysolecithin, Lecithinhydroxid und Sphingomyelin ausgewählt ist.

6. Verfahren zum Herstellen eines exzipientiafreien, gefriergetrockneten Multivitaminpräparats, bei welchem durch Solubilisieren zumindest eines fettlöslichen Vitamins mittels eines oberflächenaktiven Stoffes auf Basis von hydriertem Rhizinusöl, eines oberflächenaktiven Stoffes auf Basis eines Polyoxypropylenkondensats, vorzugsweise Polyoxalene, und eines Phospholipids, vorzugsweise einem Lecithin, einem Lecithinderivat oder einem Hauptbestandteil des Lecithins oder Sphingomyelin, in Wasser eine erste Lösung hergestellt wird, durch Auflösen zumindest eines wasserlöslichen Vitamins in Wasser eine zweite Lösung hergestellt wird, die erste Lösung und die zweite Lösung miteinander vermischt werden und das erhaltene Lösungsgemisch gefriergetrocknet wird, um ein gefriergetrocknetes Produkt zu erhalten.

7. Verfahren nach Anspruch 6, worin der oberflächenaktive Stoff auf Basis von hydriertem Rhizinusöl und/oder der oberflächenaktive Stoff auf Basis eines Polyoxypropylenkondensats und/oder das Phospholipid so wie in irgendeinem der Ansprüche 2 bis 5 definiert sind.

8. Verfahren zum Herstellen eines für parenterale Verabreichung geeigneten exzipientiafreien Multivitaminpräparats, bei welchem durch Solubilisieren zumindest eines fettlöslichen Vitamins mittels eines oberflächenaktiven Stoffes auf Basis von hydriertem Rhizinusöl, eines oberflächenaktiven Stoffes auf Basis eines Polyoxypropylenkondensats, vorzugsweise Polyoxalene, und eines Phospholipids, vorzugsweise einem Lecithin, einem Lecithinderivat oder einem Hauptbestandteil des Lecithins oder Sphingomyelin, in Wasser eine erste Lösung hergestellt wird, durch Auflösen zumindest eines wasserlöslichen Vitamins in Wasser eine zweite Lösung hergestellt wird, die erste Lösung und die zweite Lösung miteinander vermischt werden, das erhaltene Lösungsgemisch gefriergetrocknet wird, um ein gefriergetrocknetes Produkt zu erhalten, und dieses gefriergetrocknete Produkt in einer für parenterale Verabreichung geeigneten Flüssigkeit rekonstituiert wird.

9. Verfahren nach Anspruch 8, worin der oberflächenaktive Stoff auf Basis von hydriertem Rhizinusöl und/oder der oberflächenaktive Stoff auf Basis eines Polyoxypropylenkondensats und/oder das Phospholipid so wie in irgendeinem der Ansprüche 2 bis 5 definiert sind.

10. Exzipientiafreies Multivitaminpräparat mit einem Gehalt eines oberflächenaktiven Stoffes auf Basis von hydriertem Rhizinusöl und gekennzeichnet durch die Anwesenheit eines oberflächenaktiven Stoffes auf Basis eines Polyoxypropylenkondensats, vorzugsweise Polyoxalene, und eines Phospholipids, vorzugsweise eines Lecithins, eines Lecithinderivats oder eines Hauptbestandteils von Lecithin oder von Sphingomyelin.

## Revendications

1. Préparation polyvitaminée sans excipient lyophilisée, caractérisée par l'absence de tout excipient et par la présence de: un agent tensio-actif de type huile de ricin hydrogénée; un agent tensio-actif de type condensat polyoxypropylénique, de préférence un polyoxalène; et un phospholipide, de préférence une lécithine, un dérivé de lécithine ou un composant principal de la lécithine, ou la sphingomyéline.

2. Préparation polyvitaminée selon la revendication 1, dans laquelle ledit agent tensio-actif de type huile de ricin hydrogénée est formé par polymérisation par addition d'environ 30 à environ 150 moles d'oxyde d'alkylène par mole d'huile de ricin hydrogénée.

3. Préparation polyvitaminée selon la revendication 2, dans laquelle ledit agent tensio-actif de type huile de ricin hydrogénée est formé par polymérisation par addition d'environ 40 à environ 120 moles d'oxyde d'alkylène par mole d'huile de ricin hydrogénée.

4. Préparation polyvitaminée selon la revendication 3, dans laquelle ledit agent tensio-actif de type huile de ricin hydrogénée est formé par polymérisation par addition d'environ 50 à environ 100 moles d'oxyde d'éthylène par mole d'huile de ricin hydrogénée.

5. Prèparation polyvitaminée selon l'une quelconque des revendications précédentes, dans laquelle le phospholipide est choisi parmi: la lécithine de jaune d'oeuf; la lécithine de soja; la dipalmitoyl-phosphatidylcholine (DPPC); la distéaroylphosphatidylcholine (DSPC); la dipalmitoylphosphatidyl-éthanolamine (DPPE); le dipalmitoylphosphatidylinositol (DPPI); la lysolécitine; l'hydroxyde de lécithine; et la sphingomyéline.

6. Procédé pour préparer une préparation polyvitaminée lyophilisée sans excipient, qui comprend les étapes de: solubilisation dans l'eau d'au moins une vitamin liposoluble à l'aide d'un agent tensio-actif de type huile de ricin hydrogénée, un agent tensio-actif de type condensat polyoxypropylénique, de préférence un polyoxalène, et un phospholipide, de préférence une lécithine, un dérivé de lécithine ou un composant principal de lécithine ou la sphingomyéline, pour obtenir une première solution; dissolution dans l'eau d'au moins une vitamine hydrosoluble pour obtenir une seconde solution; mélange ensemble de la première et de la seconde solutions; et lyophilisation de la solution mixte formée pour obtenir un produit lyophilisé.

7. Procédé selon la revendication 6, dans lequel l'agent tensio-actif de type huile de ricin hydrogénée et/ou l'agent tensio-actif de type condensat polyoxypropylénique et/ou le phospholipide sont comme défini dans l'une quelconque des revendications 2 à 5.

8. Procédé pour préparer une préparation polyvitaminée sans excipient, appropriée à l'administration parentérale, qui comprend les étapes de: solubilisation dans l'eau d'au moins une vitamine liposoluble à l'aide d'un agent tensio-actif de type huile de ricin hydrogénée, un agent tensio-actif de type condensat polyoxypropylénique, de préférence un polyoxalène, et un phospholipide, de préférence une lécithine, un dérivé de lécithine ou un composant principal de lécithine ou la sphingomyéline, pour obtenir une première solution; dissolution dans l'eau d'au moins une vitamine hydrosoluble pour obtenir une seconde solution; mélange ensemble de la première et de la seconde solutions; lyophilisation de la solution mixte formée pour obtenir un produit lyophilisé; et reconstitution dudit produit lyophilisé dans un liquide convenant à l'administration parentérale.

9. Procédé selon la revendication 8, dans lequel l'agent tensio-actif de type huile de ricin hydrogénée et/ou l'agent tensio-actif de type condensat polyoxypropylénique et/ou le phospholipide sont comme défini dans l'une quelconque des revendications 2 à 5.

10. Préparation polyvitaminée sans excipient comprenant un agent tensio-actif de type huile de ricin hydrogénée et caractérisée par la présence de: un agent tensio-actif de type condensat polyoxypropylénique, de préférence un polyoxalène; et un phospholipide, de préférence une lécithine, un dérivé de lécithine ou un composant principal de lécithine ou la sphingomyéline.